# EUROPEAN PATENT APPLICATION

(11) **EP 2 733 087 A2**
(43) Date of publication of application: **21.05.2014**
(21) Application number: 12815151.1
(22) Date of filing: 13.07.2012
(51) Int. Cl.: B65D 75/62, B65D 77/30, A61F 13/02

(54) **PACKAGING STRUCTURE FOR PATCHES**

(30) Priority: 15.07.2011 JP 2011156771
(71) Applicant: Nitto Denko Corporation, Ibaraki-shi, Osaka 567-8680 (JP); TOA Eiyo Ltd., Tokyo 104-0032 (JP)
(72) Inventor: MATSUOKA, Kensuke, Osaka 567-8680 (JP); IWAO, Yoshihiro, Osaka 567-8680 (JP); HARIMA, Jun, Osaka 567-8680 (JP); KONNO, Masakatsu, Osaka 567-8680 (JP); AOYAGI, Kazuhiro, Osaka 567-8680 (JP); TANAKA, Tomoya, Osaka 567-8680 (JP); TOYOTA, Mikio, Saitama 3300834 (JP); AKITA, Yasuhiko, Saitama 3300834 (JP); MATSUI, Tadashi, Fukushima 9600280 (JP)
(74) Representative: Grünecker, Kinkeldey, Stockmair & Schwanhäusser
(86) International application number: PCT/JP2012/068001
(87) International publication number: WO 2013/011961

(57) **Abstract**

A purpose of the present invention is to provide a packaging structure for a patch, in which the patch can be easily taken out of a package body. The packaging structure (100) for a patch includes a first and second base materials (120, 130) which have a peripheral edge part laminated with each other, and the patch is included therein. The first and second base materials (120, 130) have a first to forth sides (121 to 124, 131 to 134). At least one of the first and second base materials (120, 130) has a first to third opening parts (125 to 127, 135 to 137). The first to third opening parts (125 to 127, 135 to 137) guides so as to open toward the inner peripheral edge (120c1, 120c2, 120c4, 130c1, 130c2, 130c4) of the sealing part (120c, 130c).

## Description

### Technical Field

The present invention relates to a packaging structure for patches, and more specifically relates to a packaging structure for patches, including a patch and a package body having the patch contained therein.

### Background Art

A patch is used to attach to a skin, thereby protecting a skin surface or transdermally administering drugs, and therefore contains a pressure-sensitive adhesive layer. The patch is required to have stability of drugs and to be stored over a long period of time. Therefore, a package body for packaging patches is important.

As such a package body, a packaging bag for transdermal absorbents is disclosed in, for example, JP-A-2008-213923 (Patent Document 1). The packaging bag disclosed in Patent Document 1 is constituted of a package material having a laminate structure of two or more layers in which a synthetic resin film is used as a base material, wherein the packaging bag is sealed by heat-sealing the front and back of at lease three sides of four sides in the state of containing the transdermal absorbent. Furthermore, in the packaging bag of Patent Document 1, an opening assisting means by the respective rows of perforations is formed in a vertically and horizontally crossing direction along the inner edge of each seal portion in a region between each sealing part of two adjacent vertical and horizontal sides and the transdermal absorbent contained.

### Prior Art Literature

### Patent Document

Patent Document 1: JP-A-2008-213923

### Summary of the Invention

### Problems that the Invention is to Solve

In the packaging bag for transdermal absorbents of Patent Document 1, the opening assisting means is formed along the sealing parts of two adjacent vertical and horizontal sides. Therefore, when the packaging bag of Patent Document 1 is opened, the two vertical and horizontal sides having the opening assisting means formed therein are opened. Because the transdermal absorbent contains a pressure-sensitive adhesive layer, the transdermal absorbent sometimes adheres to the packaging bag. For this reason, in Patent Document 1, the transdermal absorbent is sometimes difficult to be taken out of the packaging bag, depending on the place of the bag to which the transdermal absorbent is adhered.

In view of the above problems, the present invention has an object to provide a packaging structure for patches, in which the patch can be easily be taken out of a package body.

### Means for Solving the Problems

The packaging structure for a patch according to the present invention comprises: a patch, and a package body including a first base material and a second base material, which have a peripheral edge part laminated with each other, the patch being contained in an inside thereof and the inside being sealed, wherein each of the first base material and the second base material has a first side, a second side connected to the first side and intersecting with the first side, a third side connected to the second side and intersecting with the second side, and a fourth side connected to the first and third sides and intersecting with the first and third sides; each of the first base material and the second base material has a sealing part formed along the first to fourth sides so as to bond the first base material and the second base material to each other and so as to seal the inside; at least one of the first base material and the second base material has a first opening part, a second opening part and a third opening part;
the first opening part guides so as to open toward the fourth side from the second side along an inner peripheral edge of the sealing part formed along the first side in an inner peripheral side of the sealing part; the second opening part guides so as to open toward the first side from the third side along the inner peripheral edge of the sealing part formed along the second side in the inner peripheral side of the sealing part; and, in the inner peripheral side of the sealing part, the third opening part guides so as to open toward the first side from the third side along the inner peripheral edge of the sealing part formed along the fourth side, or guides so as to open toward the second side from the fourth side along the inner peripheral edge of the sealing part formed along the third side.

According to the packaging structure for patches of the present invention, the opening is possible along the first side by the first opening part, the opening is possible along the second side by the second opening part, and the opening is possible along the third side or the fourth side by the third opening part. For this reason, when taking out the patch, the opening is possible in directions along at least three sides of the four sides in the first and second base materials. Therefore, even in the case where the patch is adhered to at least one of the first and second base materials, the patch can be easily separated from the first or second base material, and as a result, the patch can be easily taken out of the package body.

In the above packaging structure for a patch, the first to third opening parts preferably include a notch.

By this, the package body can be easily opened in a direction toward a tip of the notch, and as a result, the patch can be easily taken out of the package body.

In the above packaging structure for a patch, the first to third opening parts preferably include at least one of an opening order indication part indicating the order of opening and an opening direction indication part indicating the direction of opening.

By opening the first to third opening parts according to the indication parts, the package body can be easily opened in directions along at least three sides. Therefore, the patch can be easily taken out of the package body.

In the above packaging structure for a patch, the first to third opening parts preferably contain a weakening line.

By opening along the weakening lines, the package body can be easily opened in directions along three sides. Therefore, the patch can be easily taken out of the package body.

In the above packaging structure for a patch, the patch preferably has a support and a pressure-sensitive adhesive layer, the pressure-sensitive adhesive layer being formed on at least one surface of the support and containing a drug.

By this, even in the case where the patch has a pressure-sensitive adhesive layer containing a drug, the patch can be sealed when not in use, and additionally the patch can be easily taken out of the package body when opening.

### Advantages of the Invention

As described above, according to the present invention, the packaging structure for patches, in which a patch can be easily taken out of a package body can be provided.

### Brief Description of the Drawings

FIG. 1 is a cross-sectional view schematically showing a packaging structure for patches in the embodiment 1 of the present invention, and is a view taken along I-I line in FIG. 2.
FIG. 2 is a plain view schematically showing a packaging structure for patches in the embodiment 1 of the present invention.
FIG. 3 is a cross-sectional view schematically showing a packaging structure for patches in the embodiment 1 of the present invention, and is a view taken along III-III line in FIG. 2.
FIG. 4 is a plain view schematically showing one step when a patch is taken out of a packaging structure in the embodiment 1 of the present invention.
FIG. 5 is a plain view schematically showing one step when a patch is taken out of a packaging structure in the embodiment 1 of the present invention.
FIG. 6 is a plain view schematically showing one step when a patch is taken out of a packaging structure in the embodiment 1 of the present invention.
FIG. 7 is a plain view schematically showing a packaging structure for patches in the embodiment 2 of the present invention.
FIG. 8 is a plain view schematically showing a packaging structure for patches in the embodiment 3 of the present invention.
FIG. 9 is a plain view schematically showing a packaging structure for patches in the embodiment 4 of the present invention.
FIG. 10 is a plain view schematically showing a packaging structure for patches in the embodiment 5 of the present invention.
FIG. 11 is a plain view schematically showing a packaging structure for patches in the embodiment 6 of the present invention.

### Mode for Carrying Out the Invention

Preferred embodiments of the present invention are described below, but the detailed description thereof and specific examples are intended only for the purpose of exemplification and do not limit the scope of the present invention. The description of the following preferred embodiments has merely illustrative purpose, and is not intended to limit the present invention, its utilization or its use.

Furthermore, the embodiments of the present invention are described based on the drawings, but in the following drawings, the same or corresponding parts have the same reference numerals and signs, and the description thereof is not repeated.

### (Embodiment 1)

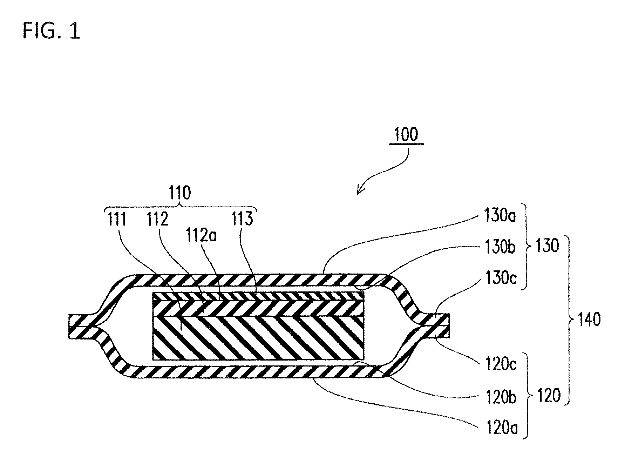

A packaging structure 100 for patches in one embodiment of the present invention is described by referring to FIGs. 1 to 3. The package structure 100 for patches is a structure in which a patch 110 is contained in the inside of a package body 140.

As shown in FIGs. 1 to 3, the packaging structure 100 for patches in the present embodiment includes the patch 110 and the package body 140. In the package body 140, the peripheral edge part of a first base material 120 and the peripheral edge part of a second base material 130 are laminated with each other, and the patch 110 is contained in the inside thereof. In other words, the patch 110 is contained in a sealed space of the package body 140 (between the first base material 120 and the second base material 130).

The patch 110 is first described by mainly referring to FIG. 1. The patch 110 includes a support 111, a pressure-sensitive adhesive layer 112 formed on at least one surface of the support 111, and a release liner 113 formed on the pressure-sensitive adhesive layer 112. The planar shape of the patch 110 is not particularly limited, and is, for example, rectangle, and the corner may be round (R may be provided), and may become angular.

The support 111 is not particularly limited. Plastic film, non-woven fabric, paper, woven fabric, knitted fabric, metal foil, or laminate of those can be used. Furthermore, those having been subjected to metal deposition can be used.

The plastic film is not particularly limited, and examples thereof include various films including a polyvinyl chloride alone; a copolymer of a monomer such as ethylene, propylene, vinyl acetate, acrylic acid, acrylic acid ester, methacrylic acid, methacrylic acid ester, acrylonitrile, styrene or vinylidene chloride, and other monomer; an olefinic polymer such as polyethylene, polyprolylene or ethylene-vinyl acetate copolymer; a polyester-based polymer such as polyethylene terephthalate (PET) or polyether polyester; or a polyamide-based polymer such as polyether polyamine block polymer.

The thickness of the support 111 is, for example, 5 µm or more and 500 µm or less, and preferably 10 µm or more and 200 µm or less.

The pressure-sensitive adhesive layer 112 has a pressure-sensitive adhesive surface 112a showing pressure-sensitive adhesiveness at an ordinary temperature (for example, 25°C) on the surface opposite the support 111, and is formed in a layer form by a pressure-sensitive adhesive having pressure-sensitive adhesiveness at an ordinary temperature (for example, 25°C).

The pressure-sensitive adhesive is not particularly limited, and examples thereof include an acrylic pressure-sensitive adhesive including an acrylic polymer; a rubber-based pressure-sensitive adhesive containing a styrene-diene-styrene block copolymer (such as a styrene-isoprene-styrene block copolymer or a styrenebutadiene-styrene block copolymer), polyisoprene, polyisobutylene, polybutadiene or the like; a silicone-based pressure-sensitive adhesive containing silicone rubber, dimethylsiloxane base, diphenylsiloxane base or the like; a vinyl ether-based pressure-sensitive adhesive such as polyvinyl methyl ether, polyvinyl ethyl ether or polyvinyl isobutyl ether; a vinyl ester-based pressure-sensitive adhesive such as a vinyl acetate-ethylene copolymer; and a polyester-based pressure-sensitive adhesive including a carboxylic acid component such as dimethyl telephthalate, dimethyl isophthalate or dimethyl phthalate, and a polyhydric alcohol component such as ethylene glycol.

In the case where the pressure-sensitive adhesive layer 112 contains a rubber-based pressure-sensitive adhesive, a tackifier such as a rosin-based resin, a polyterpene resin, a coumarone-indene resin, a petroleum-based resin, a terpene-phenol resin or a xylene resin may be contained in the pressure-sensitive adhesive layer 112 in order to impart appropriate pressure-sensitive adhesiveness thereto. Those tackifiers may be used in one kind or two or more kinds together. Examples of the petroleum-based resin include an aliphatic (C5-based) petroleum resin, an aromatic (C9-based) petroleum resin, a copolymer-based (C5-C9-based) petroleum resin, an alicyclic saturated hydrocarbon resin obtained by partially hydrogenating or completely hydrogenating an aromatic (C9-based) petroleum resin and the like.

Various organic liquid components may further be added to the pressure-sensitive adhesive layer 112. The organic liquid component contained in the pressure-sensitive adhesive layer 112 is not particularly limited, but is preferably a component having the effect of accelerating transdermal absorption. Examples of such an organic liquid component include glycols such as ethylene glycol, diethylene glycol, propylene glycol, triethylene glycol, polyethylene glycol and polypropylene glycol; oils and fats such as olive oil, castor oil, squalane and lanolin; hydrocarbons such as liquid paraffin; various surfactants; etholylated stearyl alcohol; glycerin monoesters such as oleic acid monoglyceride, caprylic acid monoglyceride and lauric acid monoglyceride; glycerin diesters, glycerin triesters or their mixtures; fatty acid alkyl esters such as ethyl laurate, isopropyl myristate, isotridecyl myristate, octyl palmitate, isopropyl palmitate, ethyl oleate and diisopropyl adipate; higher fatty acids such as oleic acid and caprylic acid; long-chain aliphatic alcohols such as a linear aliphatic alcohol such as 1-dodecanol, 1-tetradecanol or 1-hexadecanol, and a branched-chain aliphatic alcohol such as 2-hexyl-1-decanol, 2-octyl-1-dodecanol or 2-hexyl-1-tetradecanol; N-methylpyrrolidone; 1,3-butanediol and the like.

In the case where the pressure-sensitive adhesive layer 112 contains the organic liquid component, the content of the organic liquid component in the pressure-sensitive adhesive layer 112 is preferably 5% by weigh or more and 70% by weight or less, more preferably 10% by weight or more and 65% by weight or less, and most preferably 15% by weight or more and 60% by weight or less, based on the total weight of the pressure-sensitive adhesive layer 112. The thickness of the pressure-sensitive adhesive layer 112 is preferably 20 µm or more and 300 µm or less, more preferably 30 µm or more and 250 µm or less, and most preferably 50 µm or more and 200 µm or less. In the case where the pressure-sensitive adhesive layer 112 contains the organic liquid component, there is a possibility that the organic liquid component and the like protrude from the peripheral edge part of the patch 110 before use or from the cut formed in a release liner 113. However, when the content of the organic liquid component in the pressure-sensitive adhesive layer 112 and the thickness of the pressure-sensitive adhesive layer 112 fall within the above ranges, the organic liquid component and the like can be suppressed from protruding from the peripheral edge part of the patch 110 before use or from the cut formed in a release liner 113.

The pressure-sensitive adhesive layer 112 is preferably an acrylic pressure-sensitive adhesive or a rubber-based pressure-sensitive adhesive from the standpoint that soft feeling can be imparted when adhering to a skin, and is preferably an acrylic pressure-sensitive adhesive from the standpoint that the pressure-sensitive adhesive can be easily crosslinked and a large amount of the liquid component can be maintained in the pressure-sensitive adhesive layer.

Examples of the preferred acrylic pressure-sensitive adhesive include an acrylic acid ester-based pressure-sensitive adhesive including, as a main component, a polymer containing (meth)acrylic acid C₂-C₁₈ alkyl ester as a polymerization component.

Examples of the preferred rubber-based pressure-sensitive adhesive include a rubber-based pressure-sensitive adhesive including, as a main component, at least one selected from polyisobutylene, polyisoprene and a styrene-diene-styrene copolymer.

The pressure-sensitive adhesive layer 112 may further contain a drug. A patch having a pressure-sensitive adhesive layer containing a drug is sometimes particularly called a patch preparation. However, the term "patch" used herein is used in the intention to encompass such a patch preparation. In other words, the patch of the present invention includes a meaning of a patch having a pressure-sensitive adhesive layer containing a drug and a meaning of a patch having a pressure-sensitive adhesive layer that does not contain a drug.

The drug is not particularly limited, and a drug which can be transdarmally adsorbed can be used. Examples thereof include corticosteroids, an antiinflammatory analgesic, a hypnotic and sedative, a tranquilizer, an antiarrhythmic agent, a heart failure treatment agent, an antihypertensive agent, a diuretic, an antibiotic, an anesthetic, an antimicrobial agent, an antifungal agent, a vitamin compound, a vasodilator, an antihistamine, an antitussive, a sex hormone, an antidepressant, a cerebral function activator, an antiemetic, an antitumor agent and a living body medicine. Those drugs can be used in combination of two or more kinds as necessary. For example, bisoprolol or its pharmaceutically allowable salt can be used as such drugs. For example, fumarate can be used as the pharmaceutically allowable salt.

The content of those drugs can be appropriately set depending on the kind of drugs and the purpose of administration. The dugs are generally contained in the pressure-sensitive adhesive layer 112 in an amount of 0.1% by weight or more and 40% by weight or less, and preferably 0.5% by weight or more and 30% by weight or less. When the content is 0.1% or more, the release of drugs in an amount effective to treatment can be expected, and when the content is 0.5% by weight or more, it is more effective for treatment. When the content is 40% by weight or less, the effect of treatment can be developed, and additionally it is economically advantageous. When the content is 30% by weight or less, the effect of treatment can be further enhanced, and additionally it is further economically advantageous.

The release liner 113 covers a pressure-sensitive adhesive surface 112a of the pressure-sensitive adhesive layer 112. The release liner 113 protects the patch 110 until it is used.

The release liner 113 is not particularly limited, and examples thereof include plastic films such as a PET film, a polyester film other than the PET film, a polyimide film, a polypropylene film, a polyethylene film and a polycarbonate film; metal-deposited plastic films obtained by depositing metals on the plastic films; papers such as Japanese paper, Western paper, kraft paper, glassine paper and wood free paper; fibrous materials such as non-woven fabric and fabric; and metal foils.

Furthermore, laminate sheets of plastic films and papers, laminate sheets of plastic films and metal foils, and the like can be used as materials of the release liner 113.

From the standpoint that there are various kinds of industrial products, many products having a thickness suitable for use as a patch are commercially available, and a product having the required quality can be easily selected, a polyester film is preferred and a polyethylene terephthalate film is more preferred, as a material of the release liner 113.

To easily peel the release liner 113 from the pressure-sensitive adhesive layer 112, cuts may be formed on the release liner 113 and release treatment may be applied to a side contacting the pressure-sensitive adhesive surface 112a to form a light-peeling surface on the release liner 113.

The package body 140 having the patch 110 contained in the inside thereof is described below. The package body 140 includes a first base material 120 and a second base material 130, as shown in FIGs. 1 to 3. The first base material 120 and the second base material 130 are laminated and bonded at an outer peripheral side (peripheral edge part) with each other. In the present embodiment, the first base material 120 and the second base material 130 are bonded in a region containing an outer peripheral edge (by sealing parts 120c, 130c).

The first base material 120 and the second base material 130 in the present embodiment have nearly the same shape and are laminated such that the outer peripheral edges overlap to each other. The first base material 120 and the second base material 130 may have different shapes.

As shown in FIG. 1, the first base material 120 has a main surface (outer side surface) 120a, a back surface (inner side surface) 120b opposite the main surface 120a, and a sealing part 120c that has been bonded to the second base material 130. The second base material 130 has a main surface (outer side surface) 130a, a back surface (inner side surface) 130b opposite the main surface 130a, and a sealing part 130c that has been bonded to the first base material 120.

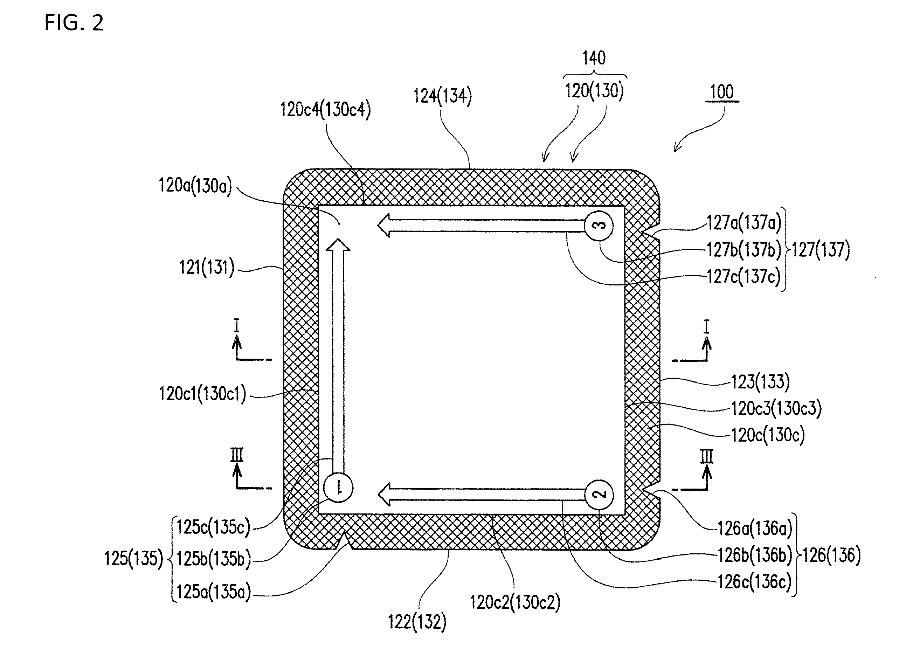

As shown in FIG. 2, the main surface 120a of the first base material 120 and the main surface 130a of the second base material 130 are that the plain shape is rectangle (square in the present embodiment). That is, the main surface 120a of the first base material 120 and the main surface 130a of the second base material 130 respectively have first sides 121, 131, second sides 122, 132 connected to the first sides 121, 131 and intersecting with the first sides 121, 131, third sides 123, 133 connected to the second sides 122, 132 and intersecting with the second sides 122, 132, and fourth sides 124, 134 connected to the first sides 121, 131 and the third sides 123, 133 and intersecting with the first sides 121, 131 and the third sides 123, 133.

Each corner of the main surfaces 120a, 130a may have roundness (R may be provided), and may be angular. The first to fourth sides 121 to 124, 131 to 134 are outer peripheral edges of the main surfaces 120a, 130a.

The sealing parts 120c, 130c are formed along the first to fourth sides 121 to 124, 131 to 134. The sealing parts 120c, 130c may contain the first to fourth sides 121 to 124, 131 to 134 and may not contain the first to fourth sides 121 to 124, 131 to 134. The sealing parts 120c, 130c of the present embodiment contain the first to fourth sides 121 to 124, 131 to 134, the outer peripheral edges are the first to fourth sides 121 to 124, 131 to 134, and the inner peripheral edges 120c1 to 120c4, 130c1 to 130c4 are parallel to the first to fourth sides 121 to 124, 131 to 134, respectively. That is, in the inner peripheral edges 120c1 to 120c4, 130c1 to 130c4 of the sealing parts 120c, 130c of the present embodiment, the plain shape is rectangle.

The inner space surrounded by the sealing parts 120c and 130c is sealed, and the patch 110 is contained therein. Therefore, the patch 110 is sealed by the sealing parts 120c and 130c. As a result, in the case that the pressure-sensitive adhesive layer 112 of the patch 110 contains a drug, the drug can be stored stably and over a long period of time.

The method for bonding the first and second base materials 120 and 130 (method for forming the sealing parts 120c, 130c) is not particularly limited, but the first and second base materials 120, 130 are preferably bonded by heat-sealing. The sealing parts 120c, 130c in this case are sealed parts. The first and second base materials 120, 130 may be connected by using, for example, a connecting member such as an adhesive. The sealing parts 120c, 130c in this case include the connecting member.

As shown in FIG. 2, at least one of the first and second base materials 120, 130 has first opening parts 125, 135 containing notches 125a, 135a formed in the second sides 122, 132, second opening parts 126, 136 containing notches 126a, 136a formed in the third sides 123, 133, and third opening parts 127, 137 containing notches 127a, 137a formed in the third sides 123, 133. In the present embodiment, the first to third opening parts 125 to 127, 135 to 137 are formed on both the first and second base materials 120, 130.

As shown in FIG. 2, the first opening part 125 (135) guides so as to open toward the fourth side 124 (134) from the second side 122 (132) along the inner peripheral edge 120c1 (130c1) of the sealing part 120c (130c) formed along the first side 121 (131) in the inner peripheral side of the sealing part 120c (130c). In other words, the first opening part 125 (135) is oriented so as to form an opening line on a non-sealing part (inner peripheral side of the sealing part 120c (130c)) along the inner peripheral edge 120c1 (130c1) of the sealing part 120c (130c) formed on the first side 121 (131).

The second opening part 126 (136) guides so as to open toward the first side 121(131) from the third side 123 (133) along the inner peripheral edge 120c2 (130c2) of the sealing part 120c (130c) formed along the second side 122 (132) in the inner peripheral side of the sealing part 120c (130c). In other words, the second opening part 126 (136) is oriented so as to form an opening line on a non-sealing part along the inner peripheral edge 120c2 (130c2) of the sealing part 120c (130c) formed on the second side 122 (132). The second opening part 126 (136) is formed nearer to the second side 122 (132) in the third side 123 (133).

The third opening part 127 (137) guides so as to open toward the first side 121 (131) from the third side 123 (133) along the inner peripheral edge 120c4 (130c4) of the sealing part 120c (130c) formed along the fourth side 124 (134) in the inner peripheral side of the sealing part 120c (130c). In other words, the third opening part 127 (137) is oriented so as to form an opening line on a non-sealing part along the inner peripheral edge 120c4 (130c4) of the sealing part 120c (130c) formed on the fourth side 124 (134). The third opening part 127 (137) is formed nearer to the fourth side 124 (134) in the third side 123 (133).

Each of the first to third opening parts 125 to 127 (135 to 137) contains the notches 125a to 127a (135a to 137a). The notch 125a (135a) of the first opening part 125 (135) has cuts formed toward the fourth side 124 (134) from the second side 122 (132) in the vicinity of the first side 121 (131) in the sealing part 120c (130c) formed in the second side 122 (132). The notch 126a (136a) of the second opening part 126 (136) has cuts formed toward the first side 121 (131) from the third side 123 (133) in the vicinity of the second side 122 (132) in the sealing part 120c (130c) formed in the third side 123 (133). The notch 127a (137a) of the third opening part 127 (137) has cuts formed toward the first side 121 (131) from the third side 123 (133) in the vicinity of the fourth side 124 (134) in the sealing part 120c (130c) formed in the third side 123 (133).

The notches 125a to 127a (135a to 137a) are cuts having a plain shape of, for example, triangle (V-shape). The shape of the notches 125a to 127a (135a to 137a) is not particularly limited, and may be linear or U-shaped cuts or depressions.

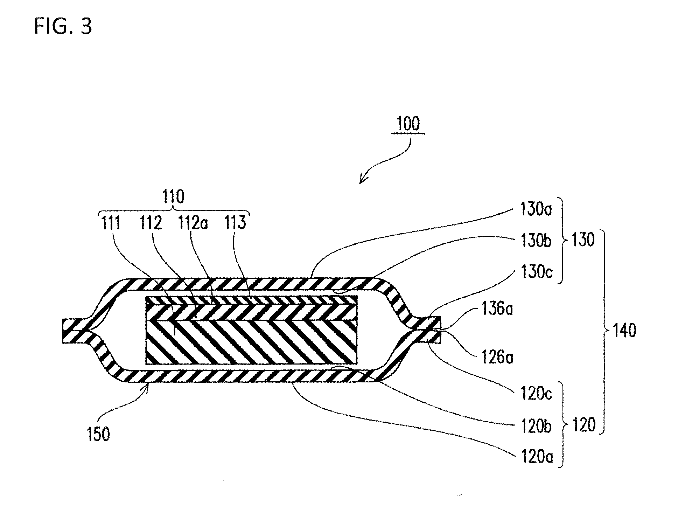

As shown in FIGs. 2 and 3, the notches 125a to 127a (135a to 137a) in the present embodiment are formed so as to pass through from the main surface 120a (130a) to the back surface 120b (130b).

The first to third opening parts 125 to 127 (135 to 137) further include opening order indication parts (hereinafter sometimes simply referred to as "indication parts") 125b to 127b (135b to 137b) indicating the order of opening. The opening order indication parts 125b to 127b (135b to 137b) are formed by printing on the package body 140, and indicate the order of opening to users. The opening order indication parts 125b to 127b (135b to 137b) are formed at the inner peripheral side of the sealing part 120c (130c) and in the vicinity of the notches 125a to 127a (135a to 137a).

The opening order indication part 125b (135b) of the first opening part 125 (135) indicates the first opening, and therefore the indication "1" is formed thereon. The opening order indication part 126b (136b) of the second opening part 126 (136) indicates the second opening, and therefore the indication "2" is formed thereon. The opening order indication part 127b (137b) of the third opening part 127 (137) indicates the third opening, and therefore the indication "3" is formed thereon.

In the present embodiment, the opening order indication part 126b (136b) of the second opening part 126 (136) has the indication "3". Therefore, the opening order indication part 127b (137b) of the third opening part 127 (137) may have the indication "2".

The first to third opening parts 125 to 127 (135 to 137) further include opening direction indication parts (hereinafter sometimes simply referred to as "indication parts") 125c to 127c (135c to 137c) indicating the direction of opening. The opening direction indication parts 125c to 127c (135c to 137c) are formed by printing on the package body 140, and indicate the direction of opening to users. The opening direction indication parts 125c to 127c (135c to 137c) are formed in the vicinity of the opening order indication parts 125b to 127b (135b to 137b).

The opening direction indication part 125c (135c) of the first opening part 125 (135) has the indication of an arrow toward the fourth side 124 (134) from the second side 122 (132) along the inner peripheral edge 120c1 (130c1) of the sealing part 120c (130c) formed along the first side 121 (131) in the inner peripheral side of the sealing part 120c (130c). The opening direction indication part 126c (136c) of the second opening part 126 (136) has the indication of an arrow toward the first side 121 (131) from the third side 123 (133) along the inner peripheral edge 120c2 (130c2) of the sealing part 120c (130c) formed along the second side 122 (132) in the inner peripheral side of the sealing part 120c (130c). The opening direction indication part 127c (137c) of the third opening part 127 (137) has the indication of an arrow toward the first side 121 (131) from the third side 123 (133) along the inner peripheral edge 120c4 (130c4) of the sealing part 120c (130c) formed along the fourth side 124 (134) in the inner peripheral side of the sealing part 120c (130c).

Materials constituting the first and second base materials 120 and 130 are described below. The materials constituting the first and second base materials 120 and 130 are not particularly limited. Examples of the materials that can be used include films or sheets including, as a main component material, polyvinyl alcohol, polyvinyl halide, polyvinyl ester, polyester, polypropylene, polyamide, polystyrene, polysulfone, polyacrylic acid ester, polymethacrylic acid ester, polyacrylonitrile or cellulose.

The first and second base materials 120 and 130 may be a single layer structure composed of only one layer, and may be a multilayer structure having two or more layers. Furthermore, the first ad second base materials 120 and 130 may be a laminate structure obtained by laminating the above material layer as an inner layer with various plastic films or metal foils, and may be package materials having an outermost layer having been subjected to metal deposition, paint coating, printing or the like. As one example, in the case of the patch 110 having a pressure-sensitive adhesive layer containing a drug, to prevent the drug contained in the patch 110 from subjecting to contamination or oxidative decomposition by air, a laminate obtained by laminating a base material with a gas-impermeable film such as a polyester film or a metal foil is preferably used as the first and second base materials 120 and 130. Furthermore, a laminate in which a polyacrylonitrile-based copolymer having a high barrier property was used as an innermost layer (innermost layer of the package material) of the first and second base materials 120 and 130 is more preferred.

The material of the first base material 120 and that of the second base material 130 may be the same, or may be different.

The thickness of the first and second base materials 120 and 130 is not particularly limited. From the standpoint of storage stability and ease of opening, the thickness is, for example, 10 µm or more and 200 µm or less, and preferably 20 µm or more and 100 µm or less.

The size of the first and second base materials 120 and 130 is not particularly limited. From the standpoint of opening operation or the standpoints of costs and productivity, the shortest distance between the facing sides is preferably 10 mm or more and 250 mm or less. The shortest distance between the facing sides means, as shown in FIG. 2, a distance between the first side 121 (131) and the third side 123 (133) (that is, length of the second and fourth sides 122 (132) and 124 (134)), and a distance between the second side 122 (132) and the fourth side 124 (134) (that is, length of the first and third sides 121 (131) and 123 (133)), excluding round portions in the case where the corner at which each side intersects has roundness.

A method for taking the patch 110 out of the package body 140 of the packaging structure 100 for patches in the present embodiment is described below by referring to FIGs. 1 to 6.

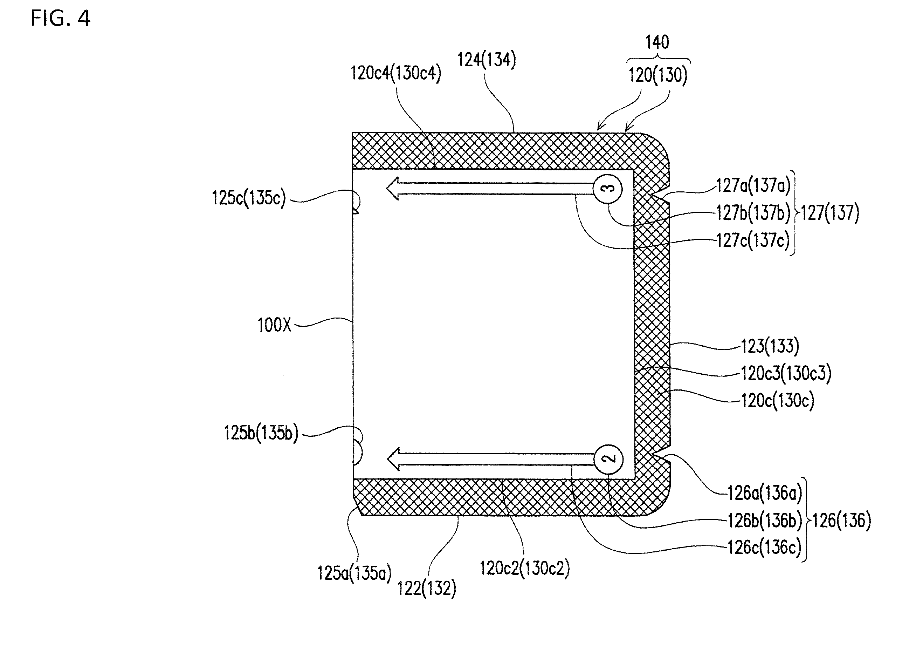

As shown in FIGs. 1 to 3, opening is conducted along the inner peripheral edge 120c1 (130c1) of the sealing part 120c (130c) formed along the first side 121 (131) in the inner peripheral side of the sealing part 120c (130c), using the notch 125a (135a) of the first opening part 125 (135). In this case, a part of the first and second base materials 120 and 130 is cut off according to the direction of an arrow indicated on the opening direction indication part 125c (135c). Because the sealing part 120c (130c) including the first side 121 (131) can be separated by this as shown in FIG. 4, an opening 100X which is opened on the side of the first side 121 (131) can be formed. When the opening 100X is formed, the space in the inside of the package body 140 becomes the state of being released in air from the sealed state.

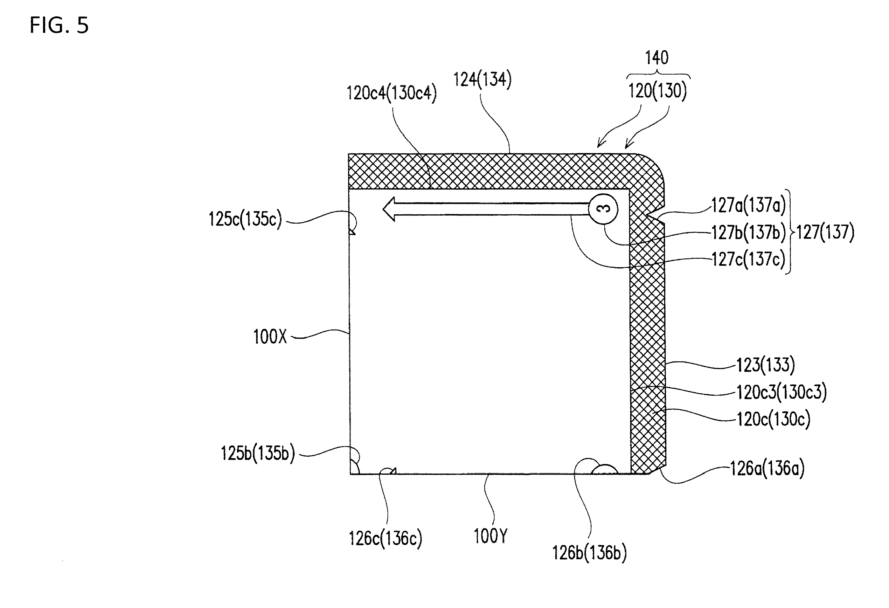

A part of the first and second base materials 120 and 130 is cut off along the inner peripheral edge 120c2 (130c2) of the sealing part 120c (130c) formed along the second side 122 (132) in the inner peripheral side of the sealing part 120c (130c), using the notch 126a (136a) of the second opening part 126 (136). In this case, the part is cut off according to the direction of an arrow indicated on the opening direction indication part 126c (136c). Because the sealing part 120c (130c) including the second side 122 (132) can be separated by this as shown in FIG. 5, an opening 100Y which is opened on the side of the second side 122 (132) can be formed.

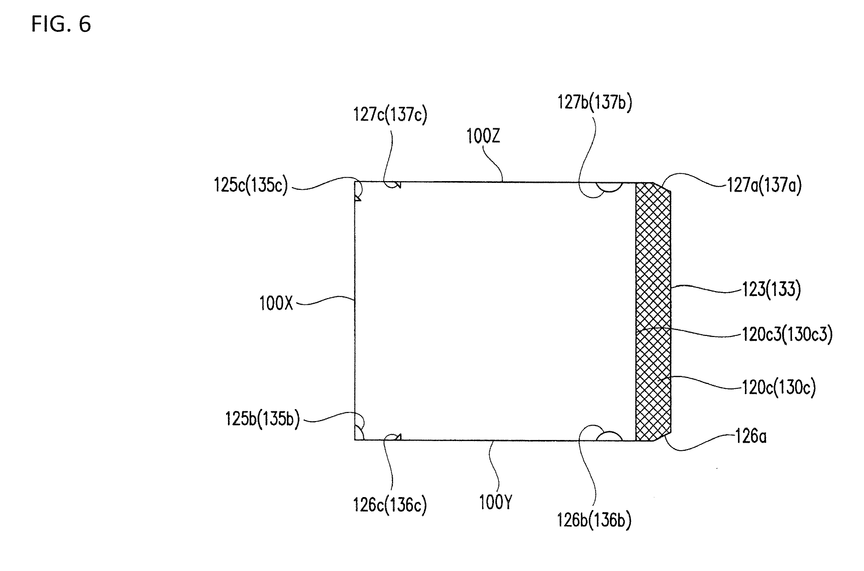

A part of the first and second base materials 120 and 130 is then cut off along the inner peripheral edge 120c4 (130c4) of the sealing part 120c (130c) formed along the fourth side 124 (134) in the inner peripheral side of the sealing part 120c (130c), using the notch 127a (137a) of the third opening part 127 (137). In this case, the part is cut off according to the direction of an arrow indicated on the opening direction indication part 127c (137c). Because the sealing part 120c (130c) including the fourth side 124 (134) can be separated by this as shown in FIG. 6, an opening 100Z which is opened on the side of the fourth side 124 (134) can be formed.

By carrying out the above steps, opening is conducted according to the directions of the first, second and fourth sides 121 (131), 122 (132) and 124 (134) of the four sides of the first and second base materials 120 and 130. By this, fingers are inserted from the openings 100X, 100Y and 100Z, and a part of the patch 110 is picked and taken out. In this case, even though components such as a pressure-sensitive adhesive oozes out of the pressure-sensitive adhesive layer 112 of the patch 110 and the patch 110 adheres to at least one inner surface (at least one of back surfaces 120b, 130b) of the first and second base materials 120 and 130 by the oozed components, the patch 110 can be easily taken out of the package body 140 by the openings 100X, 100Y and 100Z opened in the directions along three sides.

In the present embodiment, the first to third opening parts 125 to 127 (135 to 137) include the opening order indication parts 125b to 127b (135b to 137b) indicating the order of opening. As a result, opening can be easily conducted along the directions of three sides by opening in the order indicated on the opening order indication parts 125b to 127b (135b to 137b).

In the present embodiment, after opening in the direction along the second side 122 (132) by the second opening part 126 (136), opening is conducted in the direction along the fourth side 124 (134) by the third opening part 127 (137), but the order may be reverse. In other words, in the present embodiment, if the sealing part 125 (135) is first opened, three sides are opened regardless of the order of opening of the second and third opening parts 126 (136) and 127 (137).

Furthermore, in the present embodiment, opening is conducted along three sides of four sides by the first to third opening parts 125 to 127 (135 to 137), but opening may be conducted in the directions along the four sides (opening may be further conducted in the direction along the third side 123 (133)). In this case, because the openings can be provided in the directions along all of the sides, the patch 110 can be more easily taken out.

### (Embodiment 2)

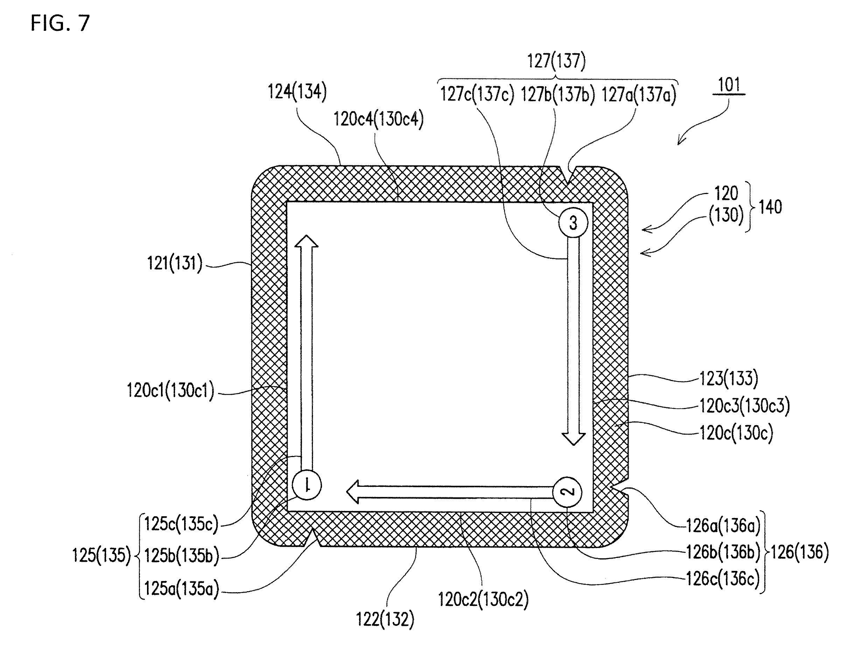

The packaging structure 101 for patches in the present embodiment is described by referring to FIG. 7. As shown in FIG. 7, the packaging structure 101 for patches in the embodiment 2 of the present invention is basically the same as the packaging structure 100 in the embodiment 1 shown in FIG. 2, but the third opening part 127 (137) differs.

Specifically, the third opening part 127 (137) in the present embodiment includes the notch 127a (137a) formed in the fourth side 124 (134), and guides so as to open toward the second side 122 (132) from the fourth side 124 (134) along the inner peripheral edge 120c3 (130c3) of the sealing part 120c (130c) formed along the third side 123 (133) in the inner peripheral side of the sealing part 120c (130c).

In the notch 127a (137a) of the third opening part 127 (137) formed in the fourth side 124 (134), a cut is formed toward the second side 122 (132) from the fourth side 124 (134) in the vicinity of the third side 123 (133) in the sealing part 120c (130c) formed in the fourth side 124 (134).

Furthermore, the third opening part 127 (137) further includes the opening order indication part 127b (137b) indicating the order of opening formed in the vicinity of the notch 127a (137a) in the inner peripheral side of the sealing part 120c (130c).

Furthermore, the third opening part 127 (137) further includes the opening direction indication part 127c (137c) indicating an arrow toward the second side 122 (132) from the fourth side 124 (134) along the inner peripheral edge 120c3 (130c3) of the sealing part 120c (130c) formed along the third side 123 (133) in the inner peripheral side of the sealing part 120c (130c).

In the present embodiment, the notches 125a (135a) to 127a (137a) of the opening parts (the first to third opening parts 125 (135) to 127 (137)) are formed in at least three sides (the second to fourth sides 122 (132) to 124 (134)), respectively, and notches of other opening parts are not provided in the vicinity of sides facing in opening directions of the opening parts 125 (135) to 127 (137).

A method for taking the patch 110 out of the package body 140 of the packaging structure 101 for patches in the present embodiment is basically the same as the embodiment 1, but a direction of opening the package body 140 by the third opening part 127 (137) differs.

Specifically, similar to the embodiment 1, opening is conducted in the directions along the first and second sides 121 (131) and 122 (132) in this order by the first and second opening parts 125 (135) and 126 (136). Opening is then conducted along the inner peripheral edge 120c3 (130c3) of the sealing part 120c (130c) formed along the third side 123 (133) by the third opening part 127 (137) in the inner peripheral side of the sealing part 120c (130c). By this, openings can be formed in the directions along the first to third sides 121 to 123 (131 to 133) of the four sides of the first and second base materials 120 and 130. As a result, the patch 110 can be easily taken out of the openings in the directions along three sides.

According to the packaging structure 101 for patches in the present embodiment, when the patch 110 is taken out of the package body 140, in the case where the first opening part 125 (135), the second opening part 126 (136) and the third opening part 127 (137) are opened in this order, the side to be cut off (non-sealing part inside the sealing part including its side) is always at right side. As a result, a right-handed user holds the package body 140 by one's left hand, picks up the side to be cut off with a right hand, and can open by pulling the picked side by a right hand. Because at least three directions of the package body 140 can be opened with only a right hand that is a dominant hand by rotating the package body 140, feel stressed when opening can be reduced.

### (Embodiment 3)

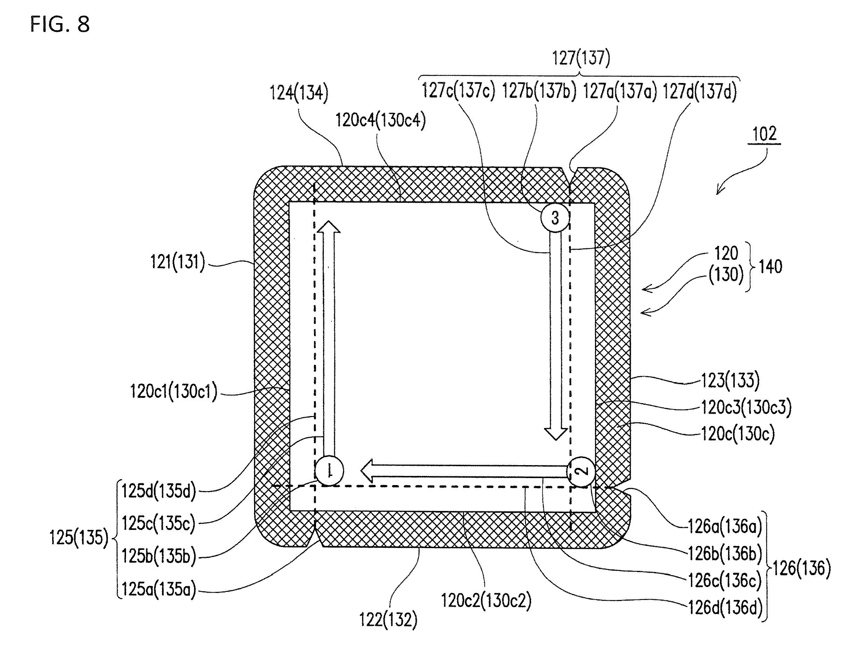

The packaging structure 102 for patches in the present embodiment is described by referring to FIG. 8. The packaging structure 102 for patches in the present embodiment is basically the same as the packaging structure 101 in the embodiment 2, but differs in that the first to third opening parts 125 (135) to 127 (137) further contain weakening lines 125d (135d) to 127d (137d).

Specifically, the weakening line 125d (135d) of the first opening part 125 (135) is linearly formed toward the fourth side 124 (134) from the tip of the notch 125a (135a). The weakening line 126d (136d) of the second opening part 126 (136) is linearly formed toward the first side 121 (131) from the tip of the notch 126a (136a). The weakening line 127d (137d) of the third opening part 127 (137) is linearly formed toward the second side 122 (132) from the tip of the notch 127a (137a).

In the present embodiment, the weakening lines 125d (135d) to 127d (137d) are formed along the opening direction indication parts 125c (135c) to 127c (137c), and additionally are formed between the opening direction indication parts 125c (135c) to 127c (137c) and the inner peripheral edges 120c1 (130c1) to 120c4 (130c4) of the sealing part 120c (130c).

The weakening line used herein means a line having weaker connection than other regions, and is, for example, a line processed so as to easily separate, such as perforations. The weakening line is formed by, for example, a method by perforations, half-cut, cut tape or the like.

At least one of the opening order indication parts 125b (135b) to 127b (137b) and the opening direction indication parts 125c (135c) to 127c (137c) may be omitted.

A method for taking the patch 110 out of the package body 140 of the packaging structure 102 for patches in the present invention is basically the same as the embodiment 2, but differs in the point of cutting at the weakening lines 125d (135d) to 127d (137d).

As described above, in the packaging structure 102 for patches in the present embodiment, the first to third opening parts 125 (135) to 127 (137) include the weakening lines 125d (135d) to 127d (137d). Because the opening directions of the first to third opening parts 125 (135) to 127 (137) can be easily guided by opening according to the weakening lines 125d (135d) to 127d (137d), the package body 140 can be easily opened. Therefore, the patch 110 can be easily taken out of the package body 140.

The first to third opening parts 125 (135) to 127 (137) of the packaging structure 102 in the present embodiment include the weakening lines 125d (135d) to 127d (137d). However, because opening may be conducted from the notches 125a (135a) to 127a (137a), the packaging structure of the present invention does not always require the weakening lines 125d (135d) to 127d (137d). The present invention differs from Patent Document 1 described before in that weakening lines can be omitted.

### (Embodiment 4)

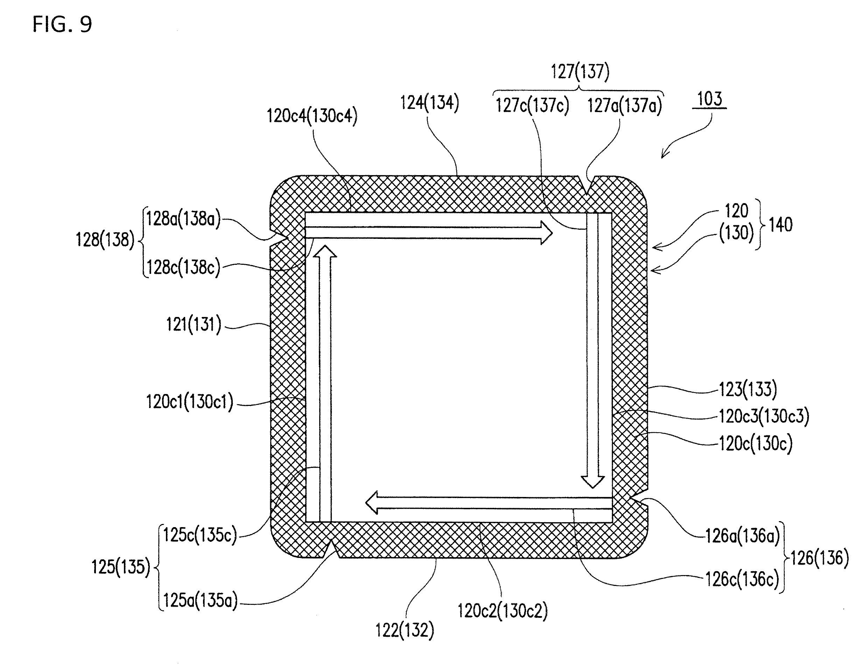

The packaging structure 103 for patches in the embodiment 4 of the present invention is described by referring to FIG. 9. The packaging structure 103 for patches of the present embodiment is basically the same as the packaging structure 101 in the embodiment 2, but differs in that at least one of the first and second base materials 120 and 130 further has the fourth opening part 128 (138).

Specifically, the fourth opening part 128 (138) guides so as to open along the inner peripheral edge 120c4 (130c4) of the sealing part 120c (130c) formed along the fourth side 124 (134) in the inner peripheral side of the sealing part 120c (130c), toward the third side 123 (133) from the first side 121 (131).

The fourth opening part 128 (138) includes a notch 128a (138a) and an opening direction indication part 128c (138c). In the notch 128a (138a) formed in the first side 121 (131), a cut is formed toward the third side 123 (133) from the first side 121 (131). The opening direction indication part 128c (138c) has the indication of an arrow toward the third side 123 (133) from the first side 121 (131) along the inner peripheral edge 120c4 (130c4) of the sealing part 120c (130c) formed along the fourth side 124 (134) in the inner peripheral side of the sealing part 120c (130c).

In the first to fourth opening parts 125 to 128 (135 to 138) in the present embodiment, the opening order indication parts indicating the order of opening are omitted.

A method for taking the patch 110 out of the packaging structure 103 in the present embodiment is basically the same as the embodiment 2, but differs in that any one of the first to fourth opening parts 125 (135) to 128 (138) may be first opened.

Specifically, firstly any one of the first to fourth opening parts 125 (135) to 128 (138) is opened along the direction indicated on the opening direction indicating parts 125c (135c) to 128c (138c). Next, of the first to fourth opening parts 125 (135) to 128 (138), the opening parts positioned in right-hand turn (clockwise rotation) are sequentially opened along the directions indicated on the opening direction indication parts. In this case, of the first to fourth opening parts 125 (135) to 128 (138), at least three opening parts are opened. By this, the package body 103 is opened in the directions along at least three sides. As a result, the patch 110 contained in the inside of the package body 140 can be easily taken out.

As described above, in at least one of the first and second base materials 120 and 130 of the packaging structure 103 for patches in the present embodiment, the notches 125a (135a) to 128a (138a) of the opening parts 125 (135) to 128 (138) are formed in all of the first to fourth sides 121 (131) to 124 (134), and notches of other opening parts are not provided in the vicinity of facing sides in the opening direction of the opening parts 125 (135) to 128 (138). Therefore, the opening part to be first opened is not particularly limited, and even thought the opening order indication part is omitted, the packaging structure 103 can be easily opened in directions along at least three sides. Therefore, the patch 110 can be easily taken out of the package body 140.

The packaging structures 101 to 103 for patches in the embodiments 2 to 4 have been described as the embodiment that is preferably used by a right-handed user, assuming the case where an indication part is formed on only one side surface (main surface 120a or main surface 130a). In the packaging structures 101 to 103 for patches of the embodiments 2 to 4, in the case where indication parts are formed on both the main surface 120a and the main surface 130a using those surfaces as mirror images, the packaging structures can be applied to a left-handed user.

### (Embodiment 5)

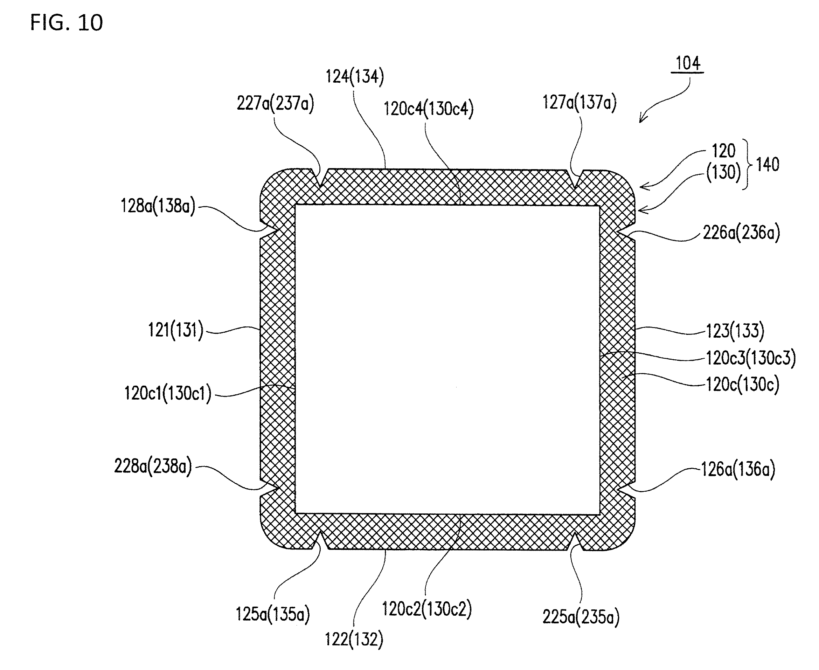

The packaging structure 104 for patches in the embodiment 5 of the present invention is described by referring to FIG. 10. The packaging structure 104 for patches in the present embodiment is basically the same as the packaging structure 103 in the embodiment 4, but differs in that the package structure further has fifth to eighth opening parts.

Specifically, the fifth to eighth opening parts in the present embodiment are notches 225a (235a) to 228a (238a).

In the second side 122 (132), the notch 125a (135a) of the first opening part is formed nearer to the first side 121 (131), and the notch 225a (235a) of the fifth opening part is formed nearer to the third side 123 (133). In other words, the notch 225a (235a) of the fifth opening part is formed in the second side 122 (132) and guides so as to open along the inner peripheral edge 120c3 (130c3) of the sealing part 120c (130c) formed along the third side 123 (133) in the inner peripheral side of the sealing part 120c (130c).

In the third side 123 (133), the notch 126a (136a) of the second opening part is formed nearer to the second side 122 (132), and the notch 226a (236a) of the sixth opening part is formed nearer to the fourth side 124 (134). In other words, the notch 226a (236a) of the sixth opening part is formed in the third side 123 (133), and guides so as to open along the inner peripheral edge 120c4 (130c4) of the sealing part 120c (130c) formed along the fourth side 124 (134) in the inner peripheral side of the sealing part 120c (130c).

In the fourth side 124 (134), the notch 127a (137a) of the third opening part is formed nearer to the third side 123 (133), and the notch 227a (237a) of the seventh opening part is formed nearer to the first side 121 (131). In other words, the notch 227a (237a) of the seventh opening part is formed in the fourth side 124 (134) and guides so as to open along the inner peripheral edge 120c1 (130c1) of the sealing part 120c (130c) formed along the first side 121 (131) in the inner peripheral side of the sealing part 120c (130c).

In the first side 121 (131), the notch 128a (138a) of the fourth opening part is formed nearer to the fourth side 124 (134), and the notch 228a (238a) of the eighth opening part is formed nearer to the second side 122 (132). In other words, the notch 228a (238a) of the eighth opening part is formed in the first side 121 (131) and guides so as to open along the inner peripheral edge 120c2 (130c2) of the sealing part 120c (130c) formed along the second side 122 (132) in the inner peripheral side of the sealing part 120c (130c).

In the present embodiment, the notch 125a (135a) of the first opening part faces the notch 227a (237a) of the seventh opening part. The notch 126a (136a) of the second opening part faces the notch 228a (238a) of the eighth opening part. The notch 127a (137a) of the third opening part faces the notch 225a (235a) of the fifth opening part. The notch 128a (138a) of the fourth opening part faces the notch 226a (236a) of the sixth opening part.

A method for taking the patch 110 out of the packaging structure 104 for patches in the present embodiment is described. The method for taking out the patch 110 is roughly classified into two methods. The first method is the same as the embodiment 4, and the package body 140 is opened using at least three of the notches 125a (135a) to 128a (138a) of the first to fourth opening parts.

The second method is that the package body 140 is opened using at least three of the notches 225a (235a) to 228a (238a) of the fifth to eighth opening parts. Specifically, any one of the notches 225a (235a) to 228a (238a) of the fifth to eighth opening parts is opened. Next, of the notches 225a (235a) to 228a (238a) of the fifth to eighth opening parts, opening parts positioned in left-hand turn (counterclockwise rotation) are sequentially opened. In this case, of the notches 125a (135a) to 128a (138a) of the fifth to eighth opening parts, at least three opening parts are opened. By this, the packaging structure is opened in the directions along at least three sides.

As described above, the packaging structure 104 for patches in the present embodiment includes the first to eighth opening parts that are formed every two parts in the first to fourth sides 121 (131) to 124 (134). Therefore, a right-handed user can open at least three directions of the package body 140 by sequentially opening the first to fourth opening parts in right-hand turn (clockwise rotation). A left-handed user can open at least three directions of the package body 140 by sequentially opening the fifth to eighth opening parts in left-hand turn (counterclockwise rotation). Therefore, because the package body 140 can be easily opened in the directions along at least three directions, regardless of the characteristic of a user, the patch 110 can be easily taken out.

### (Embodiment 6)

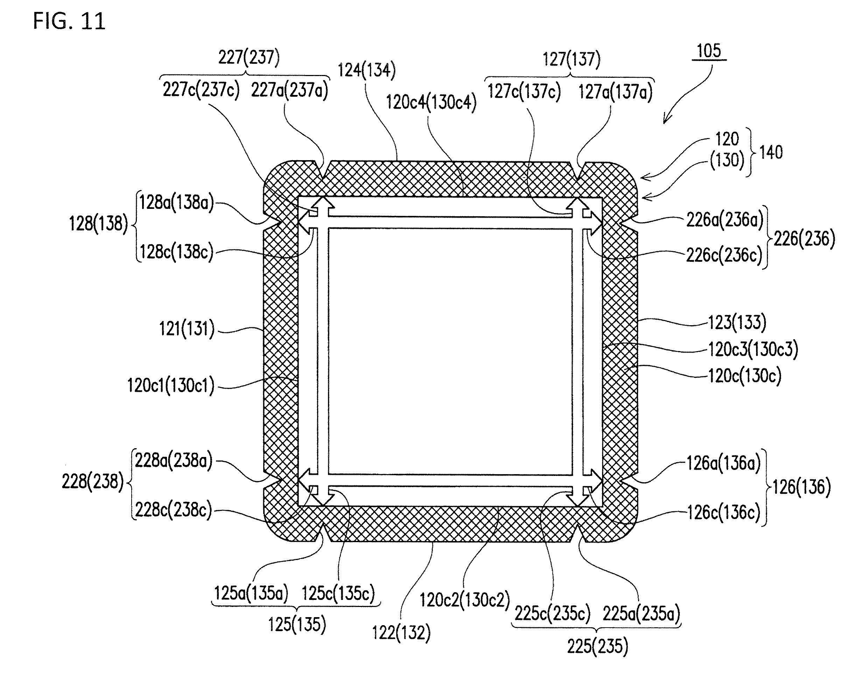

The packaging structure 105 for patches in the embodiment 6 of the present invention is described by referring to FIG. 11. The packaging structure 105 in the present embodiment is basically the same as the packaging structure 104 in the embodiment 5, but differs in that the first to eighth opening parts 125 (135) to 128 (138) and 225 (235) to 228 (238) include the opening direction indication parts 125c (135c) to 128c (138c) and 225c (235c) to 228c (238c) indicating the directions of opening.

The opening direction indication parts 125c (135c) to 128c (138c) of the first to fourth opening parts 125 (135) to 128 (138) are nearly the same as the embodiment 4, and therefore, the description thereof is not repeated. The respective fifth to eighth opening direction indication parts 225c (235c) to 228c (238c) indicate the directions opposite the opening direction indication parts 127c (137c), 128c (138c), 125c (135c) and 126c (136c) of the third, fourth, first and second opening parts 127 (137), 128 (138), 125 (135) and 126 (136).

Specifically, the opening direction indication part 225c (235c) of the fifth opening part 225 (235) has the indication of an arrow toward the fourth side 124 (134) from the second side 122 (132) along the inner peripheral edge 120c3 (130c3) of the sealing part 120c (130c) formed in the third side 123 (133) in the inner peripheral side of the sealing part 120c (130c). The opening direction indication part 226c (236c) of the sixth opening part 226 (236) has the indication of an arrow toward the first side 121 (131) from the third side 123 (133) along the inner peripheral edge 120c4 (130c4) of the sealing part 120c (130c) formed along the fourth side 124 (134) in the inner peripheral side of the sealing part 120c (130c). The opening direction indication part 227c (237c) of the seventh opening part 227 (237) has the indication of an arrow toward the second side 122 (132) from the fourth side 124 (134) along the inner peripheral edge 120c1 (130c1) of the sealing part 120c (130c) formed along the first side 121 (131) in the inner peripheral side of the sealing part 120c (130c). The opening direction indication part 228c (238c) of the eighth opening part 228 (238) has the indication of an arrow toward the third side 123 (133) from the first side 121 (131) along the inner peripheral edge 120c2 (130c2) of the sealing part 120c (130c) formed along the second side 122 (132) in the inner peripheral side of the sealing part 120c (130c).

A method for taking the patch out of the packaging structure 105 for patches in the present embodiment is the same as the embodiment 5, and therefore the description thereof is not repeated.

As described above, in the packaging structure 105 for patches in the present embodiment, the first to eighth opening parts 125 (135) to 128 (138) and 225 (235) to 228 (238) include the opening direction indication parts 125c (135c) to 128c (138c) and 225c (235c) and 228c (238c). Therefore, because the package body 140 can be easily opened in the directions along three sides, regardless of the characteristic of a user, the patch 110 can be further easily taken out.

In the packaging structure 105 for patches in the present embodiment, it is sufficient if the opening direction indication parts 125c (135c) to 128c (138c) and 225c (235c) and 228c (238c) are formed on only one side surface of the main surface 120a or the main surface 130a. The packaging structure 105 for patches in the present embodiment is advantageous in that, differing from the packaging structures 101 to 103 for patches of the embodiments 2 to 4, even in the case of forming an indication part on only one side surface, the patch 110 can be easily taken out, regardless of the characteristic of a user.

The embodiments of the present invention are described as above, but the description intends to appropriately combine the characteristic of each embodiment form the beginning. Furthermore, it should be considered that the embodiments disclosed are exemplifications in all points, and are not limitative. The scope of the present invention is shown by the scope of the claims, not the embodiments described above, and intends to include all of modifications within the sense and scope equivalent to the scope of the claims.

Although the present invention has been described in detail and by reference to the specific embodiments, it is apparent to one skilled in the art that various modifications or changes can be made without departing from the spirit and scope of the present invention. This application is based on Japanese Patent Application No. 2011-156771 filed on July 15, 2011, the disclosure of which is incorporated herein by reference.

### Description of Reference Numerals and Signs

100, 101, 102, 103, 104, 105: Packaging structure
100X, 100Y, 100Z: Opening
110: Patch
111: Support
112: Pressure-sensitive adhesive layer
112a: Pressure-sensitive adhesive surface
113: Release liner
120: First base material
120a, 130a: Main surface
120b, 130b: Back surface
120c, 130c: Sealing part
120c1 to 120c4, 130c1 to 130c4: Inner peripheral edge
121, 131: First side
122, 132: Second side
123, 133: Third side
124, 134: Fourth side
125, 135: First opening part
125a, 126a, 127a, 128a, 135a, 136a, 137a, 138a, 225a, 226a, 227a, 228a, 235a, 236a, 237a, 238a: Notch
125b, 126b, 127b, 135b, 136b, 137b: Opening order indication part
125c, 126c, 127c, 128c, 135c, 136c, 137c, 138c, 225c, 226c, 227c, 228c, 235c, 236c, 237c, 238c: Opening direction indication part
125d, 126d, 127d, 135d, 136d, 137d: Weakening line
126, 136: Second opening part
127, 137: Third opening part
128, 138: Fourth opening part
130: Second base material
140: Package body
225, 235: Fifth opening part
226, 236: Sixth opening part
227, 237: Seventh opening part
228, 238: Eighth opening part

## Claims

1. A packaging structure for a patch, comprising:
a patch, and
a package body including a first base material and a second base material, which have a peripheral edge part laminated with each other, the patch being contained in an inside thereof and the inside being sealed,
wherein each of the first base material and the second base material has a first side, a second side connected to the first side and intersecting with the first side, a third side connected to the second side and intersecting with the second side, and a fourth side connected to the first and third sides and intersecting with the first and third sides;
each of the first base material and the second base material has a sealing part formed along the first to fourth sides so as to bond the first base material and the second base material to each other and so as to seal the inside;
at least one of the first base material and the second base material has a first opening part, a second opening part and a third opening part;
the first opening part guides so as to open toward the fourth side from the second side along an inner peripheral edge of the sealing part formed along the first side in an inner peripheral side of the sealing part;
the second opening part guides so as to open toward the first side from the third side along the inner peripheral edge of the sealing part formed along the second side in the inner peripheral side of the sealing part; and
in the inner peripheral side of the sealing part, the third opening part guides so as to open toward the first side from the third side along the inner peripheral edge of the sealing part formed along the fourth side, or guides so as to open toward the second side from the fourth side along the inner peripheral edge of the sealing part formed along the third side.

2. The packaging structure for a patch according to claim 1, wherein the first to third opening parts include a notch.

3. The packaging structure for a patch according to claim 1 or 2, wherein the first to third opening parts include at least one of an opening order indication part indicating the order of opening and an opening direction indication part indicating the direction of opening.

4. The packaging structure for a patch according to any one of claims 1 to 3, wherein the first to third opening parts contain a weakening line.

5. The packaging structure for a patch according to any one of claims 1 to 4, wherein the patch has a support and a pressure-sensitive adhesive layer, the pressure-sensitive adhesive layer being formed on at least one surface of the support and containing a drug.
